# FASCICULE DE BREVET EUROPEEN

(11) **EP 2 101 849 B1**
(45) Date de publication et mention de la délivrance du brevet: **20.09.2017**
(21) Numéro de dépôt: 07871898.8
(22) Date de dépôt: 10.12.2007
(51) Int. Cl.: A61M 15/00

(54) **DISPOSITIF DE DISTRIBUTION DE PRODUIT FLUIDE**
SPENDER FÜR EIN FLÜSSIGPRODUKT
FLUID PRODUCT DISPENSING DEVICE

(30) Priorité: 11.12.2006 FR 0655406
(43) Date de publication de la demande: 23.09.2009
(73) Titulaire: Aptar France SAS, 27110 Le Neubourg (FR)
(72) Inventeur: POCOCK, Andrew Gordon, Ickleton Cambridgeshire CB10 1SX (GB); KAY, Stuart Brian William, Ickleton Cambridgeshire CB10 1SX (GB); GREENHALGH, Paul, Ickleton Cambridgeshire CB10 1SX (GB); O'HARA, Wayne, Ickleton Cambridgeshire CB10 1SX (GB)
(74) Mandataire: CAPRI
(86) Numéro de dépôt international: PCT/FR2007/052466
(87) Numéro de publication internationale: WO 2008/078032

(56) Documents cités:
- WO-A-01/26720
- WO-A-02/36189
- WO-A-03/061743

## Description

La présente invention concerne un dispositif de distribution de produit fluide, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a changé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. Un autre problème qui se pose avec les inhalateurs comportant une bande de blisters concerne le stockage de la bande, en particulier de la partie de bande comportant les réservoirs vides, une difficulté étant d'éviter les risques de blocage de la bande, sans augmenter de manière trop importante les dimensions du dispositif. WO0236189A1 divulgue les caractéristiques du préambule de la revendication 1. La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel inhalateur qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir un tel inhalateur comportant une bande de blisters dans lequel le risque de blocage de la bande de blisters est minimisé.

La présente invention a également pour but de fournir un tel inhalateur comportant une bande de blisters, dans lequel le stockage de la partie de bande comportant les réservoirs vides est amélioré.

La présente invention a donc pour objet un dispositif de distribution de produit fluide, comprenant un corps pourvu d'un orifice de distribution, ledit corps contenant une pluralité de réservoirs individuels formés sur un support commun réalisé par une bande allongée comportant une extrémité avant et une extrémité arrière, le dispositif comportant des moyens d'ouverture de réservoirs adaptés à ouvrir un réservoir respectif à chaque actionnement du dispositif, et des moyens d'entraînement de la bande allongée pour amener un réservoir plein face auxdits moyens d'ouverture avant et/ou pendant et/ou après chaque actionnement du dispositif, le dispositif comportant au moins une paroi mobile séparant au moins partiellement un logement de stockage de la bande allongée d'un logement de réception de la bande allongée, ladite bande allongée étant principalement disposée, notamment enroulée en bobine, dans ledit logement de stockage avant la première utilisation du dispositif, et se déplaçant progressivement à chaque actionnement du dispositif dans ledit logement de réception, ladite au moins une paroi mobile faisant progressivement diminuer le volume du logement de stockage et augmenter celui du logement de réception. Selon l'invention, ledit dispositif comporte deux parois mobiles pivotantes dont l'une est concave par rapport au logement de réception et l'autre concave par rapport au logement de stockage.

Avantageusement, lesdites deux parois mobiles glissent l'une contre l'autre à chaque pivotement.

Avantageusement, ladite bande allongée est disposée dans une cassette définissant lesdits logement de stockage et de réception et incorporant ladite au moins une paroi mobile.

Avantageusement, ladite au moins une paroi mobile est déplacée sous l'effet d'une poussée exercée sur elle par la bande de blisters.

Avantageusement, lesdits moyens d'ouverture comportent une aiguille fixe par rapport au corps, un réservoir étant déplacé contre ladite aiguille à chaque actionnement, ladite aiguille pénétrant à l'intérieur dudit réservoir pour le vider au moyen de l'écoulement d'inhalation.

Avantageusement, lesdits moyens d'ouverture sont actionnés par l'inhalation de l'utilisateur, de sorte que le réservoir est simultanément ouvert et vidé par ledit écoulement d'inhalation.

Ces caractéristiques et avantages et d'autres de la présente invention apparaîtront plus clairement au cours de la description détaillée suivante, faite en référence aux dessins joints, donnés à titre d'exemples non limitatifs, sur lesquels :
- la figure 1 représente une vue schématique en section transversale d'un dispositif de distribution non couvert, en début d'utilisation du dispositif ;
- la figure 2 est une vue similaire à celle de la figure 1, en fin d'utilisation du dispositif ;
- les figures 3 et 4 sont des vues schématiques en section transversale d'un dispositif de distribution non couvert, respectivement en début et en fin d'utilisation du dispositif, et
- les figures 5 et 6 sont des vues schématiques en section transversale d'un dispositif de distribution selon l'invention, respectivement en début et en fin d'utilisation du dispositif.

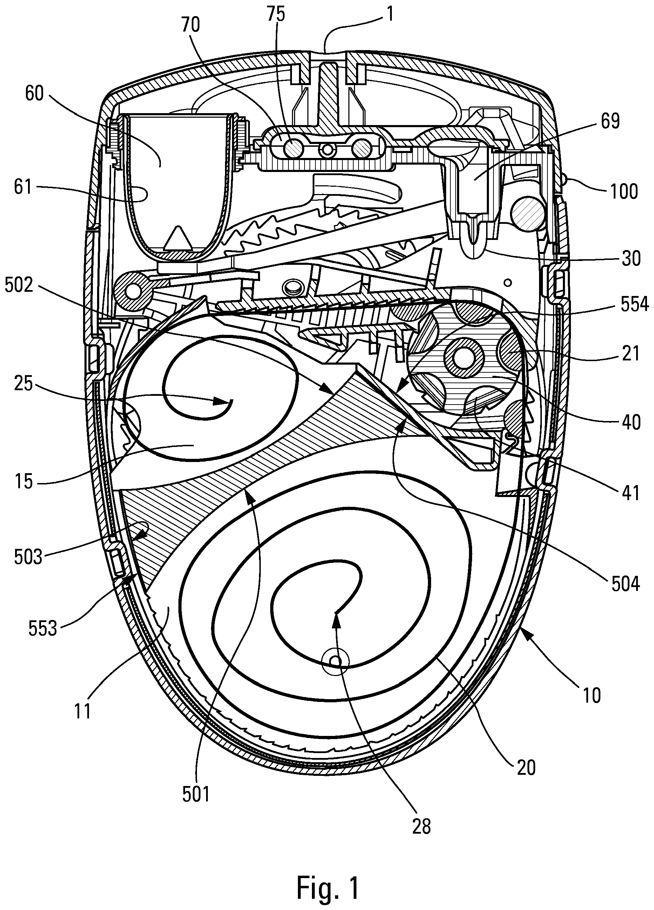

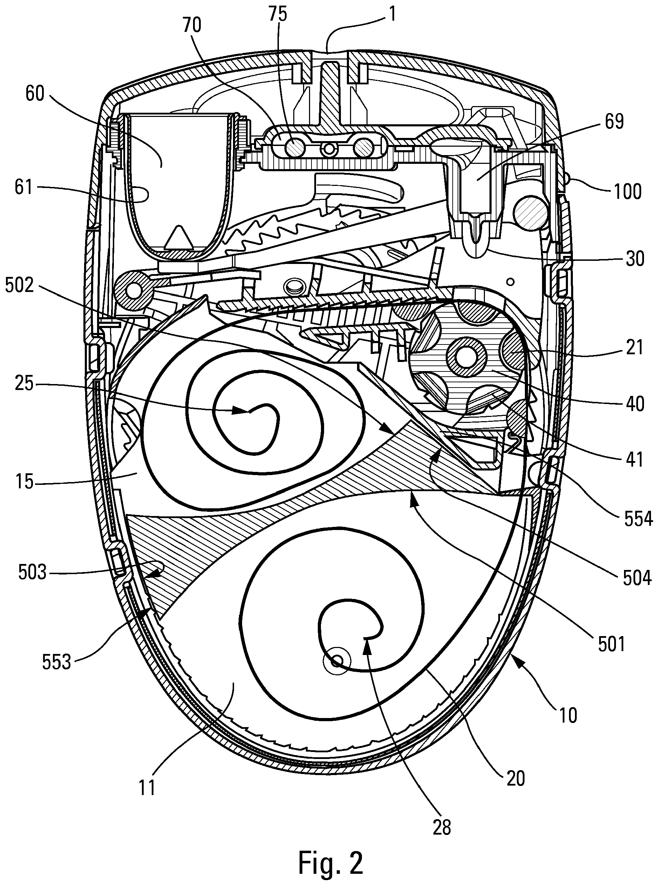

En référence aux figures 1 et 2, il est représenté un inhalateur de poudre sèche. Cet inhalateur comporte un corps 10 sur lequel peuvent être montées coulissantes une ou deux parties formant capot (non représenté) adaptées à être ouvertes pour ouvrir et charger le dispositif. Le corps 10 peut être de forme environ arrondie comme représenté sur les figures, mais il pourrait avoir tout autre forme appropriée. Le corps 10 comporte un embout buccal ou d'inhalation 1 définissant un orifice de distribution à travers lequel l'utilisateur va inhaler lors de l'actionnement du dispositif. Les capots peuvent s'ouvrir par pivotement autour d'un axe de rotation commun, mais tout autre moyen d'ouverture du dispositif est envisageable.

Le dispositif contient plusieurs réservoirs individuels 21, également appelés blisters, disposés sur un support commun formé par une bande allongée 20 sur laquelle les blisters 21 sont disposés les uns derrière les autres, de manière connue, cette bande comportant une extrémité avant 25 et une extrémité arrière 28. Ces blisters 21 ne sont pas représentés sur la totalité de la bande 20, pour ne pas surcharger le dessin dans des buts de clarté. Cette bande de blister 20 est avantageusement constituée d'une couche ou paroi de base formant les cavités 21 recevant les doses de poudre, et d'une couche ou paroi de fermeture qui obture de manière étanche chacun desdits blisters 21. Avant la première utilisation, la bande de blister 20 est enroulée à l'intérieur du corps 10, de préférence dans un logement de stockage 11, et des moyens d'entraînement de bande 40 sont prévus pour progressivement dérouler cette bande de blister et amener un réservoir individuel ou blister respectif 21 dans une position de distribution à chaque actionnement du dispositif. La partie de bande 25 comportant les réservoirs vides est adaptée à s'enrouler dans un autre endroit dudit corps 10, de préférence un logement de réception 15, comme cela sera décrit plus en détail ci-après.

L'inhalateur comporte des moyens d'ouverture de réservoir comportant un système de perçage 30 de la couche de fermeture des blisters. Par exemple, les moyens d'ouverture de réservoir comportent avantageusement un élément de perçage ou aiguille, de préférence fixe par rapport au corps 10, et contre lequel un blister respectif 21 est déplacé à chaque actionnement. Le blister 21 est alors percé par ladite aiguille qui pénètre dans ledit blister pour expulser la poudre, au moyen du flux d'inhalation de l'utilisateur.

Des moyens de déplacement 40 sont également prévus dans le dispositif, et sont adaptés à déplacer la bande de blisters 20 avant et/ou pendant et/ou après chaque actionnement du dispositif. Avantageusement, ces moyens de déplacement 40 sont aussi adaptés à déplacer le réservoir à vider 21 contre ledit système de perçage 30 lors de l'actionnement. Ces moyens de déplacement 40 peuvent être sollicités par un élément élastique, tel qu'un ressort ou tout autre élément élastique équivalent, ledit élément élastique pouvant être préchargé lors de l'ouverture du dispositif. De préférence, les moyens de déplacement 40 comportent une roue d'indexage 40 qui reçoit et guide les blisters. Une rotation de cette roue 40 fait avancer la bande de blister 20. Dans une position angulaire particulière, un réservoir donné 21 est toujours en position pour être ouvert par les moyens d'ouverture 30. Avantageusement, des moyens de positionnement en rotation de ladite roue d'indexage 40 peuvent être prévus pour déterminer précisément sa position angulaire après chaque rotation. Ces moyens de positionnement peuvent, selon une variante avantageuse, comporter une projection ou doigt dont une extrémité coopère élastiquement avec des encoches prévues autour de ladite roue 40. Éventuellement, une roue complémentaire pourrait être prévue pour aider au guidage et/ou à l'entraînement de la bande de blisters 20.

Un cycle d'actionnement du dispositif peut être le suivant. Lors de l'ouverture du dispositif, les deux parties latérales formant capot sont écartées l'une de l'autre en pivotant sur le corps pour ouvrir le dispositif, et ainsi charger le dispositif. Dans cette position la roue d'indexage 40 ne peut pas se déplacer vers l'aiguille 30 car elle est retenue par des moyens de blocage 100 appropriés. C'est lors de l'inhalation par l'utilisateur à travers l'embout buccal 1 que ces moyens de blocage sont débloqués, ce qui provoque alors le déplacement de ladite roue d'indexage 40 en direction de l'aiguille, et donc l'ouverture d'un réservoir 21.

Dans l'exemple représenté, le réservoir 21 se déplace vers sa position d'ouverture pour être ouvert par l'aiguille qui est fixe par rapport au corps. Toutefois, il est envisageable que cette aiguille puisse également être mobile pendant la phase d'ouverture du réservoir 21. Par exemple, l'aiguille pourrait se déplacer en direction du réservoir 21 pendant que le réservoir 21 se déplace en direction de l'aiguille. Dans une autre variante, on pourrait également envisager que le réservoir 21 et l'aiguille se déplacent dans la même direction lors de l'actionnement, le réservoir 21 se déplaçant plus rapidement dans cette direction de sorte qu'il vient en contact avec l'aiguille pour être ouvert.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation qui comporte avantageusement une unité 60 déplaçable et/ou déformable sous l'effet de l'inhalation, cette unité 60 étant adaptée à libérer les moyens de blocage 100. Cette unité 60 comprend avantageusement une chambre d'air déformable 61. L'inhalation de l'utilisateur provoque la déformation de ladite chambre d'air déformable 61, permettant ainsi de libérer lesdits moyens de blocage 100 et donc de permettre le déplacement de la roue d'indexage 40 et d'un réservoir respectif 21 vers sa position d'ouverture. Le réservoir 21 n'est donc ouvert qu'au moment de l'inhalation, de sorte qu'il est simultanément vidé. Il n'y a donc aucun risque de perte de dose entre l'ouverture du réservoir et son vidage.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

L'inhalateur comporte en outre une chambre de distribution 70 qui est destinée à recevoir la dose de poudre après ouverture d'un réservoir respectif 21. Avantageusement, cette chambre de distribution 70 est pourvue d'au moins une bille 75 qui se déplace à l'intérieur de ladite chambre 70 pendant l'inhalation, pour améliorer la distribution du mélange air et poudre après ouverture d'un réservoir 21, afin d'augmenter l'efficacité du dispositif.

Il peut être avantageux que le système d'ouverture 30, en particulier l'aiguille, soit formé directement sur ladite chambre de distribution 70, par exemple à l'extrémité d'un canal 69 menant à ladite chambre 70.

Après l'inhalation, lorsque l'utilisateur referme le dispositif, tous les composants reviennent vers leur position de repos initiale. Le dispositif est alors prêt pour un nouveau cycle d'utilisation.

Selon un aspect de l'invention, les réservoirs individuels ou blisters 21 sont formés sur une bande allongée 20, qui, au début, est principalement stockée sous forme de bobine dans un logement de stockage 11 à l'intérieur du corps 10 du dispositif. Avantageusement, cette bande de blister enroulée 20 peut être maintenue par des parois internes dudit logement de stockage 11 sans que son extrémité arrière 28 (dans le sens de déplacement de la bande de blisters 20) ne soit fixée par rapport audit corps 10, ce qui permet un assemblage plus aisé de cette bobine de bande de blister à l'intérieur du dispositif. La bande de blister 20 est déplacée par l'utilisateur avantageusement au moyen de la roue d'indexage 40 qui présente avantageusement au moins un, de préférence plusieurs évidements 41, dont la forme correspond à celle des blisters. Ainsi, lorsque cette roue d'indexage 40 tourne, elle entraîne la bande de blister 20. Aucun autre système d'entraînement n'est nécessaire pour déplacer la bande de blisters 20 lors de chaque actionnement. Bien entendu en variante ou de manière additionnelle, on pourrait utiliser d'autres moyens d'avancée de bande de blisters, par exemple en prévoyant un profil sur les bords longitudinaux latéraux de la bande de blister ledit profil étant adapté à coopérer avec des moyens d'entraînement appropriés. De même, des trous ménagés le long des bords latéraux de la bande de blister pourraient également être utilisés pour faire avancer la bande de blister au moyen de roues dentées coopérant avec lesdits trous.

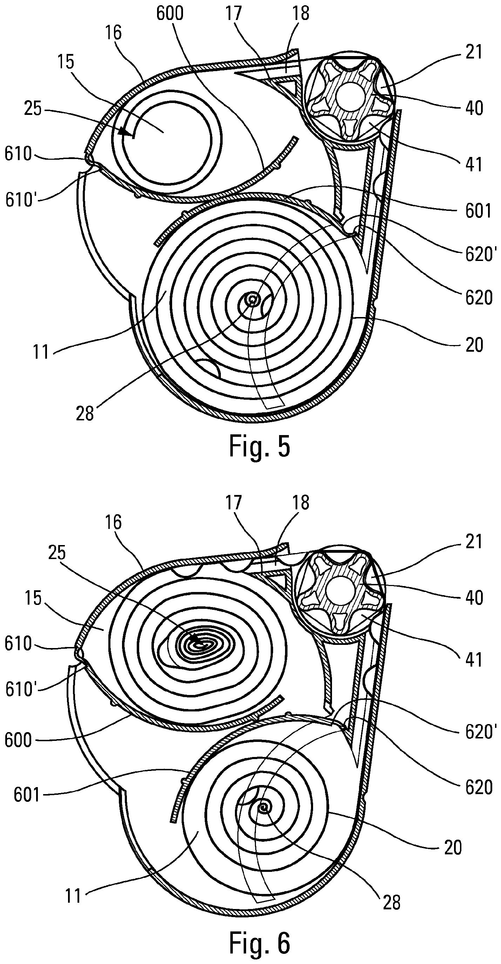

Après ouverture d'un ou plusieurs blister(s), la partie de bande de blister avec les réservoirs vidés doit pouvoir être stockée de manière aisée et non encombrante dans le dispositif. Avantageusement, cette bande de blister usée s'enroule automatiquement sur elle-même pour à nouveau former une bobine. Éventuellement, l'extrémité avant 25 de cette bande de blister 20 peut s'enrouler autour d'un élément de réception (non représenté), rotatif ou fixe. Comme visible sur les figures 5 et 6, le logement de réception 15 comporte des parois de guidage, en particulier une paroi de guidage externe 16, courbée, par exemple cylindrique, contre laquelle va glisser la bande de blisters 20. Il peut aussi y avoir une paroi de guidage interne 17 prévue à l'entrée du logement de réception 15, et s'étendant de préférence environ parallèlement à la paroi de guidage externe 16 pour former un canal de guidage 18 pour la bande de blisters 20. Ces parois de guidage 16, 17 favorisent encore davantage un bon enroulement de la bande de blisters 20. Il est prévu au moins une paroi mobile pour séparer le logement de stockage 11 du logement de réception 15, cette paroi se déplaçant au fur et à mesure des actionnements successifs du dispositif, pour progressivement augmenter le volume du logement de réception 15 et diminuer celui du logement de stockage 11. Alors qu'au début la majeure partie de la bande de blisters 20 est disposée dans le logement de stockage 11, celui-ci va progressivement se vider, alors que le logement de réception 15 va progressivement se remplir avec la partie de bande supportant les réservoirs vides. L'invention permet donc d'optimiser le volume disponible dans le corps 10. Avantageusement, c'est la bande de blisters elle-même qui va pousser ladite au moins une paroi mobile pour la déplacer à chaque actionnement.

Avantageusement, le dispositif comporte une cassette formant une unité contenant ladite bande de blisters 20, en définissant les logements de stockage 11 et de réception 15, ladite au moins une paroi mobile, et éventuellement les parois de guidage 16, 17. Cette mise en oeuvre facilite l'assemblage de la bande de blisters 20 dans le dispositif.

Les figures 1 et 2 représentent un inhalateur, dans lequel le dispositif comporte une seule paroi mobile 500, déplaçable principalement en translation. Cette paroi translative 500 comporte avantageusement deux surfaces arrondies ou concaves 501, 502 respectivement tournées vers le logement de stockage 11 et le logement de réception 15, pour s'adapter aux enroulements en bobine de la bande de blisters 20. Des surfaces de glissement latérales 503, 504 de la paroi mobile 500 sont avantageusement adaptées à glisser contre des parois de guidage complémentaires respectives 553, 554. Avantageusement, l'une ou les deux parois de guidage 553, 554 peuvent être légèrement courbées, comme visible sur les figures 1 et 2, ce qui provoque une légère rotation de la paroi mobile 500 sur elle-même pendant sa translation. Ceci permet d'optimiser encore davantage le volume disponible pour la bande de blisters 20.

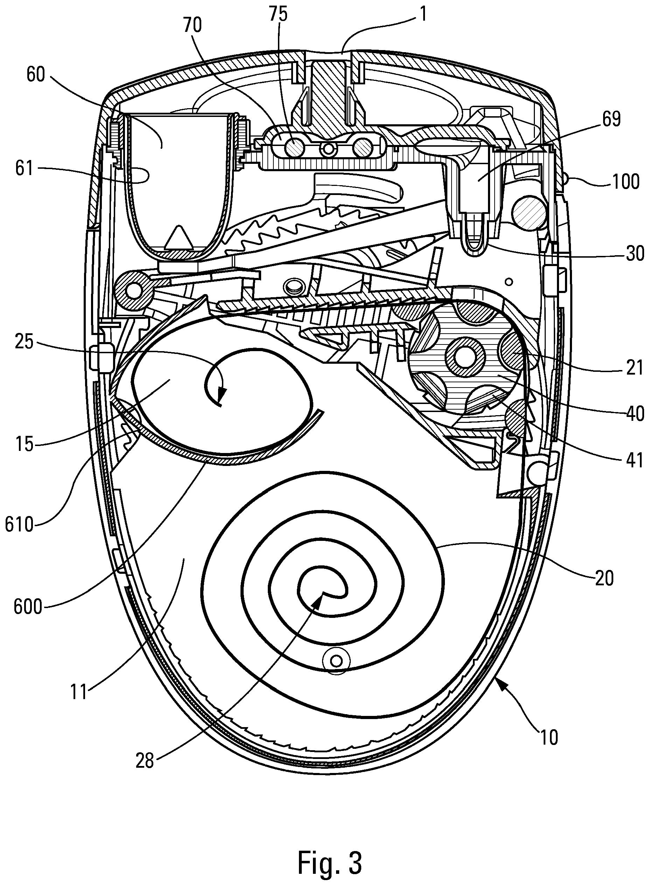

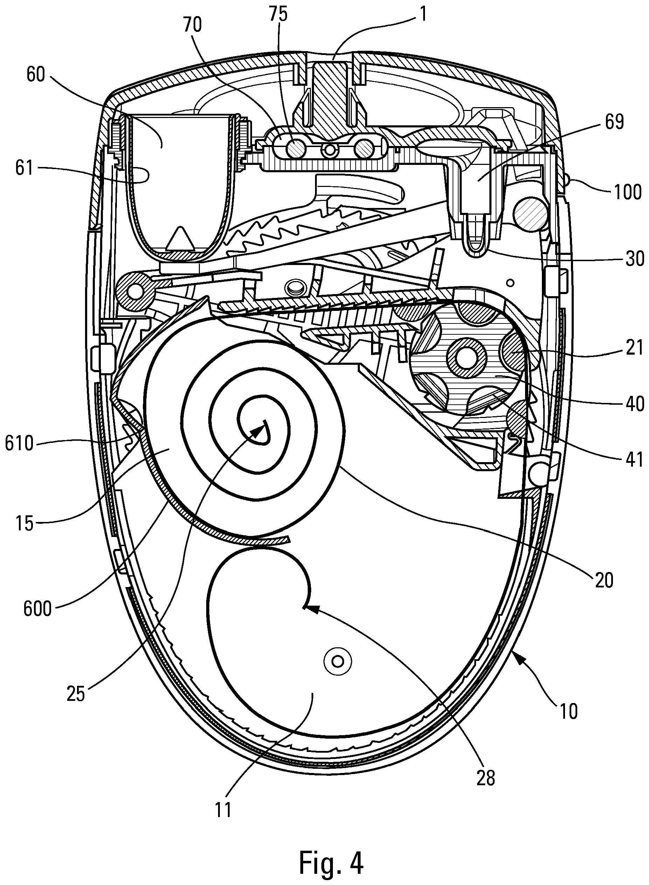

Les figures 3 et 4 montrent un second inhalateur, dans lequel il y a une seule paroi mobile 600 pivotante, avantageusement arrondie ou concave par rapport au logement de réception 15 pour s'adapter à l'enroulement en bobine de la bande de blisters 20. Cette paroi pivotante 600 peut être pivotante autour d'un point de pivotement 610 qui, comme visible sur les figures 3 et 4, peut être formé par une zone de pliage.

Les figures 5 et 6 montrent un inhalateur selon l'invention avec deux parois pivotantes 600, 601. Ici, une seconde paroi mobile 601 concave par rapport au logement de stockage 11 est prévue, les deux parois 600, 601 glissant l'une sur l'autre au niveau de leurs surfaces extérieures respectives. Avantageusement, chaque paroi pivotante 600, 601 peut être pivotante autour de deux points de pivotement 610, 610', respectivement 620, 620'. Pour chaque paroi pivotante, ces deux points de pivotement peuvent être séparés par une liaison souple, comme visible sur les figures 5 et 6. Ceci facilite le pivotement des parois et permet d'optimiser l'espace disponible.

La présente invention permet donc de fournir un inhalateur de poudre sèche qui procure notamment les fonctions suivantes :
▪ une pluralité de doses individuelles de poudre stockées dans des réservoirs individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération préchargé ;
▪ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
▪ un stockage sûr, fiable et peu encombrant de la bande de blisters.

D'autres fonctions sont également fournies par le dispositif de l'invention tel que cela a été décrit précédemment. Il est à noter que les différentes fonctions, même si elles ont été représentées comme prévues simultanément sur les différents modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les réservoirs individuels sont arrangés les uns par rapport aux autres, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées.

## Revendications

1. Dispositif de distribution de produit fluide, comprenant un corps (10) pourvu d'un orifice de distribution (1), ledit corps (10) contenant une pluralité de réservoirs individuels (21) formés sur un support commun réalisé par une bande allongée (20) comportant une extrémité avant (25) et une extrémité arrière (28), le dispositif comportant des moyens d'ouverture (30) de réservoirs adaptés à ouvrir un réservoir respectif à chaque actionnement du dispositif, et des moyens d'entraînement (40) de la bande allongée (20) pour amener un réservoir plein face auxdits moyens d'ouverture avant et/ou pendant et/ou après chaque actionnement du dispositif, **caractérisé en ce que** le dispositif comporte au moins une paroi mobile (500; 600, 601) séparant au moins partiellement un logement de stockage (11) de la bande allongée (20) d'un logement de réception (15) de la bande allongée (20), ladite bande allongée (20) étant principalement disposée, notamment enroulée en bobine, dans ledit logement de stockage (11) avant la première utilisation du dispositif, et se déplaçant progressivement à chaque actionnement du dispositif dans ledit logement de réception (15), ladite au moins une paroi mobile (500) faisant progressivement diminuer le volume du logement de stockage (11) et augmenter celui du logement de réception (15), ledit dispositif comportant deux parois mobiles pivotantes (600, 601) dont l'une (600) est concave par rapport au logement de réception (15) et l'autre concave par rapport au logement de stockage (11).

2. Dispositif selon la revendication 1, dans lequel lesdites deux parois mobiles (600, 601) glissent l'une contre l'autre à chaque pivotement.

3. Dispositif selon l'une quelconque des revendications précédentes, dans lequel ladite bande allongée (20) est disposée dans une cassette définissant lesdits logement de stockage (11) et de réception (15) et incorporant ladite au moins une paroi mobile (500; 600, 601).

4. Dispositif selon l'une quelconque des revendications précédentes, dans lequel ladite au moins une paroi mobile (500 ; 600, 601) est déplacée sous l'effet d'une poussée exercée sur elle par la bande de blisters (20).

5. Dispositif selon l'une quelconque des revendications précédentes, dans lequel lesdits moyens d'ouverture (30) comportent une aiguille fixe par rapport au corps (10), un réservoir (21) étant déplacé contre ladite aiguille (30) à chaque actionnement, ladite aiguille (30) pénétrant à l'intérieur dudit réservoir (21) pour le vider au moyen de l'écoulement d'inhalation.

6. Dispositif selon l'une quelconque des revendications précédentes, dans lequel lesdits moyens d'ouverture (30) sont actionnés par l'inhalation de l'utilisateur, de sorte que le réservoir est simultanément ouvert et vidé par ledit écoulement d'inhalation.

## Patentansprüche

1. Ausgabevorrichtung für ein fluides Produkt, aufweisend einen Körper (10), der mit einer Ausgabeöffnung (1) versehen ist, wobei der Körper (10) mehrere einzelne Behälter (21) aufweist, die auf einem gemeinsamen Träger ausgebildet sind, der durch einen langgestreckten Streifen (20) gebildet ist, der ein vorderes Ende (25) und ein hinteres Ende (28) aufweist, wobei die Vorrichtung Behälteröffnungsmittel (30), die dazu geeignet sind, bei jeder Betätigung der Vorrichtung einen jeweiligen Behälter zu öffnen, und Mitnahmemittel (40) des langgestreckten Streifens (20) aufweist, um vor und/oder während und/oder nach jeder Betätigung der Vorrichtung einen vollen Behälter in Position gegenüber den Öffnungsmitteln zu bringen, **dadurch gekennzeichnet, dass** die Vorrichtung mindestens eine bewegliche Wand (500; 600, 601) aufweist, die zumindest teilweise einen Lagerraum (11) des langgestreckten Streifens (20) von einem Aufnahmeraum (15) des langgestreckten Streifens (20) trennt, wobei der langgestreckte Streifen (20) vor der ersten Verwendung der Vorrichtung in erster Linie insbesondere auf einer Rolle aufgewickelt in dem Lagerraum (11) angeordnet ist und sich bei jeder Betätigung der Vorrichtung in dem Aufnahmeraum (15) allmählich verschiebt, wobei die mindestens eine bewegliche Wand (500) das Volumen des Lagerraums (11) allmählich verkleinert und dasjenige des Aufnahmeraums (15) vergrößert, wobei die Vorrichtung zwei bewegliche, verschwenkbare Wände (600, 601) aufweist, von denen eine (600) in Bezug auf den Aufnahmeraum (15) konkav ist und die andere in Bezug auf den Lagerraum (11) konkav ist.

2. Vorrichtung nach Anspruch 1, wobei die zwei beweglichen Wände (600, 601) bei jeder Verschwenkung gegeneinander gleiten.

3. Vorrichtung nach einem der vorhergehenden Ansprüche, wobei der langgestreckte Streifen (20) in einer Kassette angeordnet ist, die den Lagerraum (11) und den Aufnahmeraum (15) definiert und die mindestens eine bewegliche Wand (500; 600, 601) beinhaltet.

4. Vorrichtung nach einem der vorhergehenden Ansprüche, wobei die mindestens eine bewegliche Wand (500; 600, 601) unter der Wirkung eines auf diese durch den Blisterstreifen (20) ausgeübten Drucks verschoben wird.

5. Vorrichtung nach einem der vorhergehenden Ansprüche, wobei die Öffnungsmittel (30) eine Nadel (30) aufweisen, die in Bezug auf den Körper (10) befestigt ist, wobei bei jeder Betätigung ein Behälter (21) gegen die Nadel verschoben wird, wobei die Nadel in den Behälter (21) eindringt, um ihn mittels der Inhalationsströmung zu leeren.

6. Vorrichtung nach einem der vorhergehenden Ansprüche, wobei die Öffnungsmittel (30) durch Inhalation des Benutzers betätigt werden, so dass der Behälter durch die Inhalationsströmung gleichzeitig geöffnet und geleert wird.

## Claims

1. A fluid dispenser device including a body (10) that is provided with a dispenser orifice (1), said body (10) containing a plurality of reservoirs (21) formed on a common substrate formed by an elongate strip (20) that includes a leading end (25) and a trailing end (28), the device further including reservoir-opening means (30) that are adapted to open a respective reservoir each time the device is actuated, and drive means (40) for driving the elongate strip (20), so as to bring a full reservoir into register with said opening means before and/or during and/or after each actuation of the device, said device being **characterized in that** it includes at least one movable wall (500; 600, 601) that separates, at least in part, a storage housing (11) for storing the elongate strip (20) from a reception housing (15) for receiving the elongate strip (20), said elongate strip (20) being disposed, in particular rolled up in a roll, mainly in said storage housing (11) before the device is used for the first time, and being displaced progressively in said reception housing (15) each time the device is actuated, said at least one movable wall (500) causing the volume of the storage housing (11) to decrease progressively, and the volume of the reception housing (15) to increase progressively, said device including two pivot walls (600, 601) one of which (600) is concave relative to the reception housing (15) and the other one of which (601) is concave relative to the storage housing (11).

2. A device according to claim 1, in which said two pivot walls (600, 601) slide one against the other on each pivot.

3. A device according to any preceding claim, in which said elongate strip (20) is disposed in a cassette that defines said storage and reception housing (11, 15) and that incorporates said at least one movable wall (500; 600, 601).

4. A device according to any preceding claim, in which said at least one movable wall (500; 600, 601) is displaced under the effect of thrust exerted thereon by the blister strip (20).

5. A device according to any preceding claim, in which said opening means (30) comprise a needle that does not move relative to the body (10), a reservoir (21) being displaced against said needle (30) each time the device is actuated, said needle (30) penetrating inside said reservoir (21) so as to empty it by means of an inhalation flow.

6. A device according to any preceding claim, in which said opening means (30) are actuated by the user inhaling, such that the reservoir is opened and emptied simultaneously by said inhalation flow.
